**Europäisches Patentamt**

**European Patent Office**

**Office européen des brevets**

(11) Publication number: **0 108 431**
**B1**

(12) **EUROPEAN PATENT SPECIFICATION**

(45) Date of publication of patent specification: **15.06.88**

(21) Application number: **83201328.8**

(22) Date of filing: **16.09.83**

(51) Int. Cl.⁴: **G 01 K 1/12, G 01 K 7/04,**
**G 01 K 7/12, G 01 N 33/20**

(54) **Immersion measuring probe for measurements in fluid metals.**

(30) Priority: **08.10.82 BE 2059866**

(43) Date of publication of application:
**16.05.84 Bulletin 84/20**

(45) Publication of the grant of the patent:
**15.06.88 Bulletin 88/24**

(84) Designated Contracting States:
**AT DE FR GB IT LU NL SE**

(56) References cited:
**EP-A-0 045 535**
**DE-A-1 573 233**
**DE-A-2 263 469**
**DE-A-2 321 768**
**DE-A-2 501 569**
**DE-A-2 754 284**
**DE-A-3 022 189**
**FR-A-2 123 698**

The file contains technical information
submitted after the application was filed and
not included in this specification

(73) Proprietor: **ELECTRO-NITE INTERNATIONAL N.V.**
**Amerikalei 110**
**B-2000 Antwerpen (BE)**

(72) Inventor: **Cure, Omer**
**Havenlaan 8**
**B-3610 Diepenbeek (BE)**
Inventor: **Bollen, Theo Pieter Clement**
**Kneippstraat 72**
**B-3600 Genk (BE)**

(74) Representative: **Donné, Eddy**
**M.F.J.Bockstael Arenbergstraat 13**
**B-2000 Anvers (BE)**

## Description

The present invention covers an immersion measuring probe for measurements in fluid metals.

For measurement of e.g. temperature and/or oxygen content in a fluid metal, immersion probes are mostly used, which traditionally consist of a tube whose end to be dipped in first is shut off by a plug-shaped measuring head of fireproof material, carrying one or several measuring elements as well as a corresponding connector. The measuring elements protrude from the dipping side of the measuring head and the connector from the other side.

For use, a measuring probe of this type is slid over a metal supporting lance, so that the connector contacts a contact block fitted in the lance for this purpose. This contact block is connected to a measuring instrument by electric conductors passing through the lance.

The protruding portions of the measuring elements are usually protected by cardboard and/or metal caps fitted on the measuring head, which decompose, respectively melt down when dipped in the fluid metal.

The aforementioned tubes are mostly made of cardboard. Such tubes are often provided with an insulating protective sheathing to prevent splashing and vibration when immersed.

Immersion measuring probes of the type outlined above are described for example in EP—A2—0 045 535, in Belgian patents No. 828.752, No. 881.886, No. 884,405, and No. 889.276 and in the German patent application 2 501 569. Measuring elements which can be placed on top of a measuring head are described e.g. in German patent application No. 1573233, No. 2263469, No. 2 321 768 and No. 2 754 284.

An aim at still more accurate measuring and economy has induced the Applicant to develop a new generation of immersion measuring probes.

Directions were set as follows:

a) a considerable reduction of the mass in the vicinity of the measuring elements to diminish their cooling effect on the metal and thus enabling more accurate measuring;

b) if the probe comprises a thermo-couple, reduction of a temperature difference between its cold joints during immersion, respectively during measurement;

c) if the probe comprises an electro-chemical cell, minimizing the probe's own influence on the oxygen concentration to be measured and the chemical balances related thereto;

d) for economical reasons, elimination of the aforementioned insulating protection of the cardboard tube;

e) the complete protection of said aforesaid connector.

These objectives are met according to the invention as claimed by claim 1 by the measuring head consisting mainly of a metal tube, surrounded over the major part of its length by a sheathing of fireproof, respectively heat-resistant material, attached to the tube, the outer surface of this sheathing being tapered towards the metal tube end to be dipped in first, which is shut off by a plug of fireproof material carrying one or several measuring elements, while the other end of the measuring head, respectively the metal tube, is slid into one end of a tube made of cardboard or similar material.

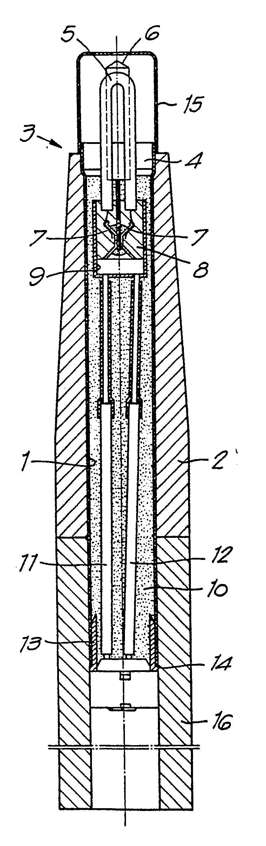

For better explanation an embodiment of the invention is described in detail below, with reference to the attached drawing showing an axial section of a measuring probe, provided with a measuring head as described before.

The measuring head shown mainly consists of a metal tube 1, surrounded over the major part of its length by a sheathing 2 fastened to tube 1 and made of moulding sand for example.

The outer surface of sheathing 2 is tapered towards the end 3 of tube 1 to be dipped first.

This end 3 is sealed by a plug 4 of fireproof cement carrying one or several measuring elements, in this case a thermo-couple 5 partly fitted in a quartz tube and an electrochemical cell 6.

The cold joints 7 of thermo-couple 5 are embedded in a gas-tight filling such as silicone 8, surrounded by a small plastic casing 9 with a cloverleaf-shaped cross section.

Tube 1 is filled with resin-impregnated but gas-permeable moulding sand 10, containing the open end of the electro-chemical cell and through which conductors 11 and 12 pass to a connector 13 shutting off the other end 14 of tube 1.

Tube 1 is used as a guide to contact the molten metal, e.g. for closing the circuit of the electrochemical cell 6.

An intrinsically known slag cap 15 is provided. A cardboard tube 16 is slid around end 14 of tube 1 until it contacts sheathing 2.

Dimensions of sheathing 2 are by way of example—but also in preference—the following: length: 10 cm; smallest external diameter (end 3): 2.5 cm; largest external diameter 3.7 to 4.8 cm; internal diameter: 1.8 cm.

The advantages of such a construction are obvious:

—extremely small mass of materials in the vicinity of the measuring elements;

—exceptional protection of cold joints against mutual temperature differences;

—favourable shape as regards penetration into the metal;

—complete protection of the connector as well as of the supporting lance, even in case of breakage of sheathing 2;

—fairly simple, cheap and none-the-less sturdy construction.

## Claims

1. Immersion measuring probe for measurements in fluid metals, of the type consisting of a tube (16) whose immersion end is shut off by a measuring head carrying at one end one or several measuring elements (5—6) as well as a corresponding connector (13) at the other end, the said tube (16) being meant to be slid over the end of a hollow supporting lance, characterised in that the measuring head mainly consists of a metal tube (1), surrounded over the major part of its length by a sheathing (2) of fireproof, respectively heat-resistant material, attached to the metal tube (1), the outer surface of said sheathing (2) being tapered towards the metal tube end (3) to be dipped first, which is shut off by a plug (4) of fireproof material carrying said measuring elements (5, 6), the other end of the measuring head, respectively of the metal tube (1), being slid into the end of first said tube (16) made of cardboard or similar material.

2. Immersion measuring probe according to claim 1, characterised in that the metal tube (1) is filled with resin-impregnated but gas-permeable mould sand (10), through which extend one or several conductors (11—12) from the said measuring element to the other end of the metal tube (1) which is shut off by a connector (13).

3. Immersion measuring probe according to claim 2, wherein a measuring element is a thermo-couple (5), characterised in that the cold joints (7) of the thermo-couple (5) are embedded in a gas-tight filling such as silicone (8) which, in turn, is embedded in moulding sand.

4. Immersion measuring probe according to claim 2 or 3, characterised in that a measuring element is an electro-chemical cell (6) with one open end, and that this open end extends into the aforementioned gas-permeable filling.

5. Immersion measuring probe according to claim 1, characterised in that the said metal tube (1) is used as a conductor, respectively for contacting the fluid metal.

**Patentansprüche**

1. Tauchmeßfühler für Messungen in flüssigen Metallen des Type bestehend aus einem Rohr (16), dessen Eintauchende verschlossen ist durch einen Meßkopf, der an einem Ende sowohl ein oder mehrere Meßelemente (5, 6) als auch ein übereinstimmendes Verbindungsstück (13) am anderen Ende trägt, wobei das Rohr (16) über das Ende einer hohlen Traglanze schieben soll, dadurch gekennzeichnet, daß der Meßkopf zur Hauptsache aus einem Metallrohr (1) besteht, das über den größeren Teil seiner Länge mit einem am Metallrohr (1) befestigten Mantel (2) aus feuerfestem oder hitzbeständigem Material umgeben ist, wobei die Außenoberfläche dieses Mantels (2) dem zuerste einzutauchenden Metallrohrende (3) konisch zuläuft, das durch einen Pfropfen (4) aus feuerfestem Material verschlossen ist, der die Meßelemente (5, 6) trägt, während das andere Ende des Meßkopfes oder des Metallrohres (1) in das Ende des ersten, aus Pappe oder ähnlichem Material hergestellten Rohres (16) hineingeschoben ist.

2. Tauchmeßfühler nach Anspruch 1, dadurch gekennzeichnet, daß das Metallrohr (1) mit harzgetränktem, aber gasdurchlässigem Formsand (10) gefüllt ist, durch den ein oder mehrere Stromleiter (11, 12) vom Meßelement zum anderen Ende des Metallrohres (1) laufen, das durch ein Verbindungsstück (13) verschlossen ist.

3. Tauchmeßfühler nach Anspruch 2, in dem ein Meßelement ein Thermoelement (5) ist, dadurch gekennzeichnet, daß die kalten Enden (7) des Thermoelementes (5) in einem gasdichten Füllmittel wie einem Silikon (8) eingebettet sind, das der Reihe nach in Formsand eingebettet ist.

4. Tauchmeßfühler nach Anspruch 2 oder 3, dadurch gekennzeichnet, daß das Meßelement eine elektrochemische Zelle (6) mit einem offenen Ende ist, und das letzteres sich im obenerwähnten gasdurchlässigen Füllmittel ausstreckt.

5. Tauchmeßfühler nach Anspruch 1, dadurch gekennzeichnet, daß das Metallrohr (1) als Stromleiter bzw. zum Berühren des flüssigen Metalls verwendet wird.

**Revendications**

1. Sonde de mesure à immersion en vue de pratiquer des mesures dans des métaux liquides, cette sonde étant du type constitué d'un tube (16) dont l'extrémité d'immersion est fermée par une tête de mesure comportant, à une extrémité, un ou plusieurs éléments de mesure (5—6), ainsi qu'un raccord correspondant (13) à l'autre extrémité, ce tube (16) étant conçu pour glisser pardessus l'extrémité d'une lance support creuse, caractérisé en ce que la tête de mesure est constituée principalement d'un tube métallique (1) entouré sur la majeure partie de sa longueur, d'une gaine (2) de matière ignifuge ou résistant à la chaleur, fixée à ce tube métallique (1), la surface extérieure de cette gaine (2) allant en diminuant en cône vers l'extrémité (3) du tube métallique qui doit être immergée en premier lieu et qui est obturée par un bouchon (4) en une matière ignifuge supportant les éléments de mesure précités (5, 6), l'autre extrémité de la tête de mesure ou du tube métallique (1) étant introduite par glissement dans l'extrémité du type (16) mentionné en premier lieu et réalisé en carton ou en une matière semblable.

2. Sonde de mesure à immersion selon la revendication 1, caractérisée en ce que le tube métallique (1) est rempli de sable de moulage (10) imprégné de résine, mais perméable aux gaz et à travers lequel s'étend ou s'étendent un ou plusieurs conducteurs (11—12) allant de l'élement de mesure précité à l'autre extrémité de ce tube métallique (1), lequel est fermé par un raccord (13).

3. Sonde de mesure à immersion selon la revendication 2, dans laquelle un élément de mesure est un thermocouple (5), caractérisée en ce que les joints froids (7) du thermocouple (5) sont enrobés dans une charge étanche aux gaz telle qu'une silicone (8) qui, à son tour, est enrobée dans du sable de moulage.

4. Sonde de mesure à immersion selon la revendication 2 ou 3, caractérisée en ce qu'un élément de mesure est une cellule électrochimique (6) comportant une extrémité ouverte, et en ce que cette extrémité ouverte s'étend à l'intérieur de la charge précitée perméable aux gaz.

5. Sonde de mesure à immersion selon la revendication 1, caractérisée en ce que le tube métallique (1) est utilisé comme conducteur ou pour établir une mise en contact avec le métal liquide.